# EUROPEAN PATENT SPECIFICATION

(11) **EP 4 206 680 B1**
(45) Date of publication and mention of the grant of the patent: **20.11.2024**
(21) Application number: 21871366.7
(22) Date of filing: 15.09.2021
(51) Int. Cl.: G01N 35/10, G01N 30/24, G01N 35/00, B01L 3/00, B01L 9/06, C12M 3/06, F25D 23/12, G01N 15/06, C12M 1/42

(54) **CONTROL METHOD FOR MICROFLUIDIC TESTING SYSTEM**
STEUERUNGSVERFAHREN FÜR MIKROFLUIDISCHES TESTSYSTEM
PROCÉDÉ DE COMMANDE POUR SYSTÈME DE TEST MICROFLUIDIQUE

(30) Priority: 27.09.2020 CN 202011029709
(43) Date of publication of application: 05.07.2023
(73) Proprietor: Qingdao Haier Refrigerator Co., Ltd., Laoshan District Qingdao Shandong 266101 (CN); Haier Smart Home Co., Ltd., Qingdao, Shandong 266101 (CN)
(72) Inventor: ZHAO, Bintang, Qingdao, Shandong 266101 (CN); FEI, Bin, Qingdao, Shandong 266101 (CN); LIU, Haoquan, Qingdao, Shandong 266101 (CN)
(74) Representative: Ziebig Hengelhaupt Intellectual Property Attorneys Patentanwaltskanzlei PartGmbB
(86) International application number: PCT/CN2021/118505
(87) International publication number: WO 2022/062995

(56) References cited:
- EP-A1- 4 206 649
- CN-A- 109 917 147
- CN-A- 111 044 742
- CN-A- 111 289 762
- CN-U- 205 538 654
- CN-U- 207 611 061
- CN-U- 214 039 110
- CN-U- 214 039 111
- CN-U- 214 039 171
- CN-U- 214 039 172
- CN-U- 214 039 173
- CN-U- 214 041 434
- US-A1- 2003 017 085
- US-A1- 2007 154 895
- US-A1- 2011 189 052
- US-A1- 2018 341 825
- US-B1- 6 406 893

## Description

### FIELD OF THE INVENTION

The present invention relates to refrigerating and freezing technologies, and particularly to a control method for a microfluidic testing system.

### BACKGROUND OF THE INVENTION

With the improvement of the living standard of people, pesticide residues, viruses, nutritional elements or other aspects of some edible food materials are usually required to be tested in daily life, so as to qualitatively or quantitatively obtain the conditions of the food materials. For example, due to the pesticide abuse problem, fruits, vegetables and agricultural and sideline products purchased daily by people may have the problem of excessive pesticide residue content, and if the problem of excessive pesticide residue content of the foods cannot be found in time, great harm may be caused after they are ingested by people. For another example, currently advocated breast feeding is best feeding for infants only when breast milk has normal nutritional value, but in cases of diseases, medicine taking, surgery or other cases of the mother, the milk secreted by the mother may have reduced content of nutritional elements and even produce viruses, thereby affecting the growth and health of the infants.

Prior art US 2003/017085 A1 provides improved systems, devices, and methods for analyzing a large number of sample compounds contained in standard multi-well microtiter plates or other array structures. The multi-well plates travel along a conveyor system to a test station having a microfluidic device. At the test station, each plate is removed from the conveyor and the wells of the multi-well plate are sequentially aligned with an input port of the microfluidic device. After at least a portion of each sample has been input into the microfluidic channel system, the plate is returned to the conveyor system. Pre and/or post testing stations may be disposed along the conveyor system, and the use of an X-Y-Z robotic arm and novel plate support bracket allows each of the samples in the wells to be input into the microfluidic network through a probe affixed to a microfluidic chip. A clamshell structure having a hinged lid can releasably support the chip while providing and/or accommodating the electrical, optical, structural, and other interface connections between the microfluidic device and the surrounding system.

US 2011/189052 A1 teaches a system for the automated analysis of liquid samples having one or more processing units for reaction between the samples and one or more reagents to thereby obtain reaction products. Disclosed also are a sample unit for supplying the samples to the one or more processing units; a reagent unit equipped with plural reagent vessels containing one or more reagents for mixing with the samples; a distribution unit for distributing fluids including the one or more reagents provided with plural distribution lines, at least some of which are connected to the reagent vessels and the one or more processing units; and at least one analytical unit for analyzing the samples based on the reaction products, in which the analytical unit may include at least one detector for detecting the reaction products.

Among testing methods, the method for testing by using a microfluidic biochip is rapid, the size is small, and the method is suitable for household use. However, sample loading of the existing microfluidic biochip is required to be manually operated by a user, and use is quite troublesome; or a sample liquid is required to be delivered to the microfluidic biochip by means of a complicated sample liquid delivering device, and the structure and control logic are quite complicated.

### BRIEF DESCRIPTION OF THE INVENTION

In summary, the independent claim 1 defines the scope of the invention and the dependent claims 2-10 defines preferred implementations of the invention.

An object of a first aspect of the present invention is to overcome at least one of the drawbacks of the prior art, and to provide a control method for a microfluidic testing system, which facilitates sample loading and has a high automation degree.

A further object of the first aspect of the present invention is to automatically prepare a sample liquid to improve the accuracy of the concentration and quantity of the sample liquid.

Another object of the first aspect of the present invention is to improve the automation degree thereof to enhance the user experience.

An object of a second aspect of the present invention is to provide a microfluidic testing system operating according to any one of the above-mentioned control methods.

An object of a third aspect of the present invention is to provide a refrigerator having a microfluidic testing system operating according to any one of the above-mentioned methods.

In the control method for a microfluidic testing system according to the present invention, the sample stage is automatically controlled to move from the initial position to the testing position after the sample cup holding the sample liquid is placed on the sample stage; at the testing position, the sample liquid in the sample cup is in contact with the sample inlet of the microfluidic biochip, thereby realizing sample loading of the microfluidic biochip. A user only needs to place the sample cup onto the sample stage, and no other operations are needed, thus, the sample loading operation is convenient, the degree of automation is high, the method is time-saving and labor-saving, and the usage experience of the user is improved.

Further, in the control method according to the present application, before the sample stage is controlled to move, the buffer liquid is automatically injected into the sample cup when the sample cup storing the sample is placed on the sample stage, and is mixed with the sample to generate the sample liquid, thus omitting the process that the user manually prepares the sample liquid, avoiding the problem that the quantity or concentration of the sample liquid is not well controlled due to manual preparation of the sample liquid, improving the accuracy of the concentration and quantity of the sample liquid, and laying a foundation for the accuracy of a testing result.

Further, in the control method according to the present application, whether the microfluidic biochip is mounted is automatically detected before the sample cup is placed on the sample stage, and if no, the prompt information is sent to prompt the user to mount the microfluidic biochip, such that the user can conveniently and rapidly mount the microfluidic biochip before the sample cup is placed. Further, in the control method according to the present application, whether other articles (for example, a sample cup left in a previous test or other articles placed on the sample stage by the user) exist on the sample stage is automatically detected before detecting whether the microfluidic biochip is mounted, and if yes, the prompt information for emptying the sample stage is sent out, such that the user can perform operations according to the prompt information, thus improving the automation degree of the microfluidic testing system, and improving the use experience of the user.

According to the following detailed description of specific embodiments of the present invention in conjunction with drawings, those skilled in the art will better understand the aforementioned and other objects, advantages and features of the present invention.

### BRIEF DESCRIPTION OF THE DRAWINGS

Some specific embodiments of the present invention will be described below in detail in an exemplary rather than restrictive manner with reference to the drawings. Identical reference numerals in the drawings represent identical or similar components or parts. Those skilled in the art should understand that these drawings are not necessarily drawn to scale. In the drawings:
Fig. 1 is a schematic structural diagram of a microfluidic testing system according to an embodiment of the present invention;
Fig. 2 is a schematic flow diagram of a control method according to a first embodiment of the present invention;
Fig. 3 is a schematic flow diagram of a control method according to a second embodiment of the present invention;
Fig. 4 is a schematic flow diagram of a control method according to a third embodiment of the present invention;
Fig. 5 is a schematic flow diagram of a control method according to a fourth embodiment of the present invention;
Fig. 6 is a schematic flow diagram of a control method according to a fifth embodiment of the present invention;
Fig. 7 is a schematic flow diagram of a control method according to a sixth embodiment of the present invention;
Fig. 8 is a schematic flow diagram of a control method according to a seventh embodiment of the present invention;
Fig. 9 is a schematic flow diagram of testing of a microfluidic biochip in an embodiment of the present invention;
Fig. 10 is a schematic sectional diagram of a microfluidic biochip suitable to be used in the method of the present invention;
Fig. 11 is a schematic flow diagram of testing of a microfluidic biochip in another embodiment of the present invention;
Fig. 12 is a schematic flow diagram of testing of a testing pool by a testing mechanism in an embodiment of the present invention; and
Fig. 13 is a schematic structural diagram of a refrigerator not forming part of the present invention.

### DETAILED DESCRIPTION

The present application firstly provides a control method for a microfluidic testing system, which is used for qualitatively or quantitatively testing a preset testing parameter of a sample liquid. The preset testing parameter may be, for example, a pesticide residue parameter for indicating whether a pesticide residue content exceeds the standard and/or a specific value of the pesticide residue content, a nutrient parameter for indicating whether a nutritional element meets the standard and/or a specific content of the nutritional element, a specific substance parameter for indicating whether a specific harmful substance (for example, a specific virus) exceeds the standard and/or a specific content thereof, or the like.

The control method for a microfluidic testing system can be generally divided into two parts of sample loading and testing. First, the sample loading part is specifically described below.

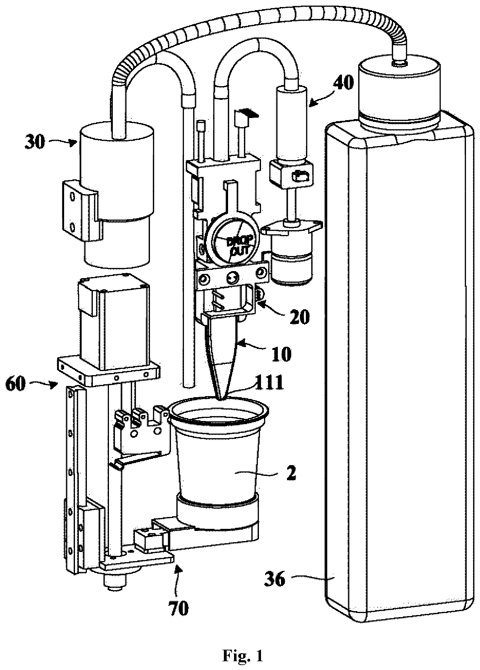

Fig. 1 is a schematic structural diagram of the microfluidic testing system suitable to be used according the method of the present invention, and for ease of understanding, a sample cup 2 is also shown in Fig. 1. The microfluidic testing system 1 includes a microfluidic biochip 10, a sample stage 70 for placing the sample cup, and a lifting mechanism 60 for driving the sample stage to move, and the microfluidic biochip 10 is provided with a sample inlet 111 for receiving a sample liquid.

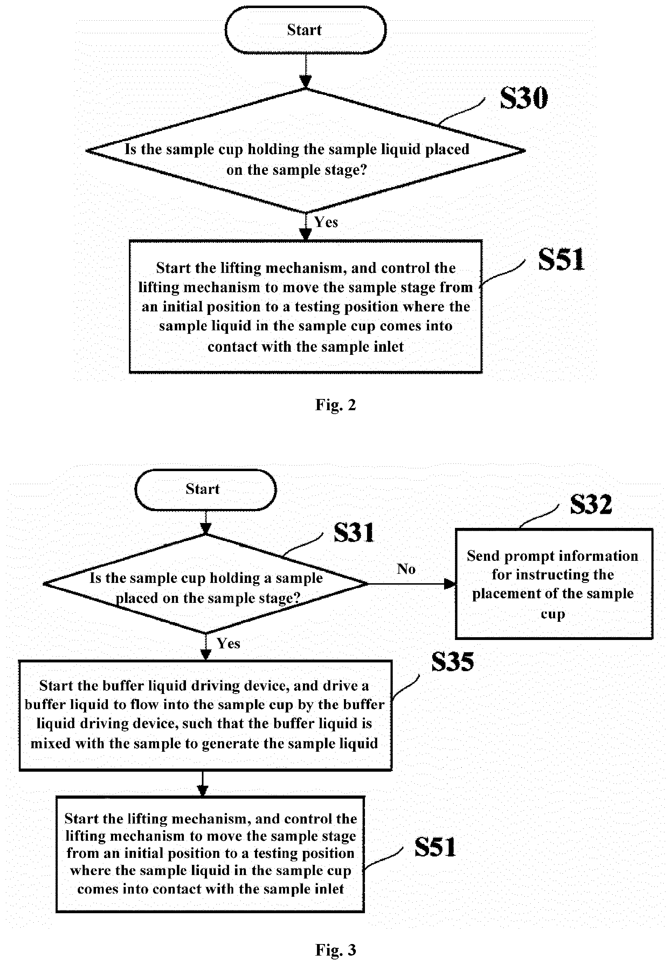

Fig. 2 is a schematic flow diagram of the control method not forming part of the present invention; referring to Fig. 2, the control method includes: starting the lifting mechanism 60 when the sample cup holding the sample liquid is placed on the sample stage 70, and controlling the lifting mechanism 60 to move the sample stage 70 from an initial position to a testing position where the sample liquid in the sample cup comes into contact with the sample inlet 111.

That is, the control method may include the following steps:
step S30: judging whether the sample cup holding the sample liquid is placed on the sample stage 70; if yes, proceeding to step S51; and
step S51: starting the lifting mechanism 60, and controlling the lifting mechanism 60 to move the sample stage 70 from an initial position to a testing position where the sample liquid in the sample cup comes into contact with the sample inlet 111.

It can be understood that when the sample stage 70 is located at the initial position, a certain distance exists between the sample cup and the sample inlet 111, and the sample liquid in the sample cup cannot be in contact with the sample inlet 111. A user places the sample cup on the sample stage 70 when the sample stage 70 is located at the initial position, so as to avoid structural interference with the microfluidic biochip 10 or inconvenient placement of the sample cup when the user places the sample cup on the sample stage 70. Specifically, the sample liquid in the sample cup may be manually added into the sample cup by the user, or automatically prepared in the sample cup by the microfluidic testing system. The sample liquid can be a to-be-tested liquid, a liquid obtained by diluting the to-be-tested liquid, a liquid obtained by dissolving a to-be-tested substance on a solid sample into a buffer liquid, a liquid obtained by mashing a food material with relatively high water content, or the like.

In the control method for a microfluidic testing system the lifting mechanism 60 is automatically controlled to move the sample stage 70 from the initial position to the testing position after the sample cup holding the sample liquid is placed on the sample stage 70; at the testing position, the sample liquid in the sample cup is in contact with the sample inlet 111 of the microfluidic biochip 10, thereby realizing sample loading of the microfluidic biochip 10. A user only needs to place the sample cup onto the sample stage, and no other operations are needed, thus, the sample loading operation is convenient, the degree of automation is high, the method is time-saving and labor-saving, and the usage experience of the user is improved.

According to the invention, the microfluidic testing system 1 further includes a buffer liquid driving device 30. Fig. 3 is a schematic flow diagram of the control method according to the present invention. Referring to Fig. 3, the step S30 of judging whether the sample cup holding the sample liquid is placed on the sample stage 70 -may specifically includes:
step S31: judging whether the sample cup holding a sample is placed on the sample stage 70; if yes, proceeding to step S35, and it may additionally include, if no, proceeding to step S32;
step S32: sending prompt information for instructing the placement of the sample cup; and
step S35: starting the buffer liquid driving device 30, and driving a buffer liquid to flow into the sample cup by the buffer liquid driving device 30, such that the buffer liquid is mixed with the sample in the sample cup to generate the sample liquid. Thus, it may be considered that the sample cup holding the sample liquid is placed on the sample stage 70. Specifically, the buffer liquid driving device 30 may be communicated with a buffer liquid bottle 36, so that the buffer liquid is supplied to the buffer liquid driving device 30 through the buffer liquid bottle 36.

That is, in the control method according to the present application, before the sample stage 70 is controlled to move, the buffer liquid is automatically injected into the sample cup when the sample cup storing the sample is placed on the sample stage 70, and is mixed with the sample to generate the sample liquid. That is, after the buffer liquid is mixed with the sample, a to-be-tested substance on the sample is dissolved into the buffer liquid to form the sample liquid, thus omitting the process that the user manually prepares the sample liquid, avoiding the problem that the quantity or concentration of the sample liquid is not well controlled due to manual preparation of the sample liquid, improving the accuracy of the concentration and quantity of the sample liquid, and laying a foundation for the accuracy of a testing result.

Specifically, in step S31, whether the sample cup holding the sample is placed on the sample stage 70 may be judged by judging whether the weight of the article borne on the sample stage 70 is within a preset range. For example, when the weight of the article borne on the sample stage 70 is zero, it is considered that no article is placed on the sample stage 70. When the weight of the article borne on the sample stage 70 is greater than a second preset weight value and less than a third preset weight value, it is considered that only an empty sample cup is placed on the sample stage 70. When the weight of the article borne on the sample stage 70 is greater than a third preset weight value and less than a fourth preset weight value, it is considered that the sample cup holding the sample is placed on the sample stage 70. When no article is placed on the sample stage 70 or only the empty sample cup is placed on the sample stage 70, the prompt information for prompting the placement of the sample may be sent.

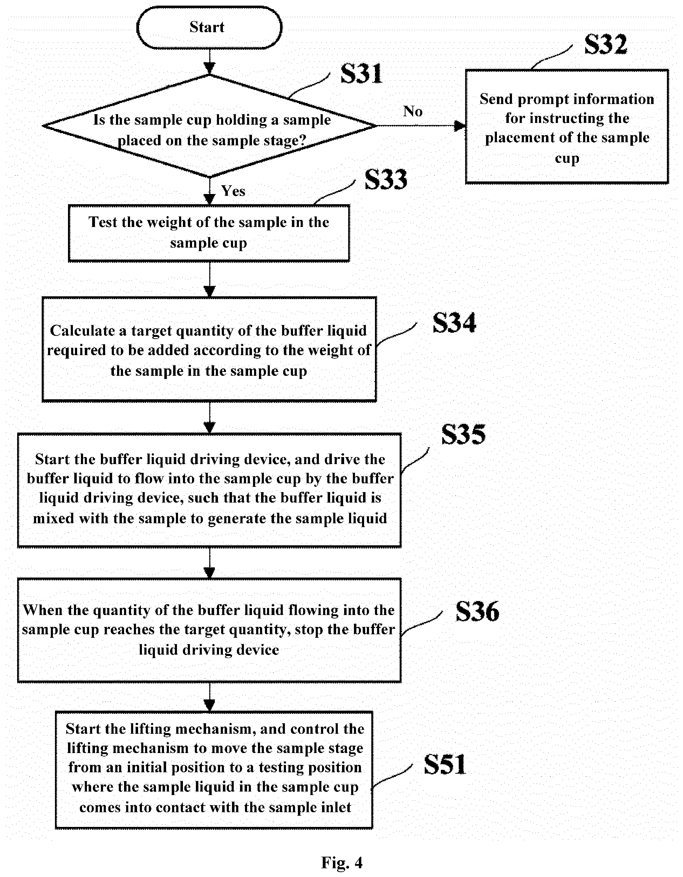

Fig. 4 is a schematic flow diagram of the control method according to a third embodiment of the present invention. Referring to Fig. 4, in some embodiments, after the sample cup holding the sample is placed on the sample stage 70 and before the buffer liquid driving device is started, the control method according to the present invention further includes:
step S33: testing the weight of the sample in the sample cup; and
step S34: calculating a target quantity of the buffer liquid required to be added according to the weight of the sample in the sample cup; and
after starting the buffer liquid driving device, the control method according to the present invention further includes:
   step S36: when the quantity of the buffer liquid flowing into the sample cup reaches the target quantity, stopping the buffer liquid driving device. Specifically, the quantity of the buffer fluid flowing into the sample cup can be tested and controlled by testing the total weight on the sample stage.

It may be appreciated that, in general, the sample is extracted at will by the home user, for example, a small vegetable leaf is torn off at will, and therefore, in order to guarantee the accuracy of a measurement result, the quantity of the buffer liquid input into the sample cup is required to be matched with the quantity of the sample, so as to generate the sample liquid with a proper concentration. In the present application, the weight of the sample is automatically obtained, and the buffer liquid with the target quantity is automatically calculated and output according to the weight of the sample, such that the user can conveniently take the sample at will, and the accuracy of the testing result can be guaranteed.

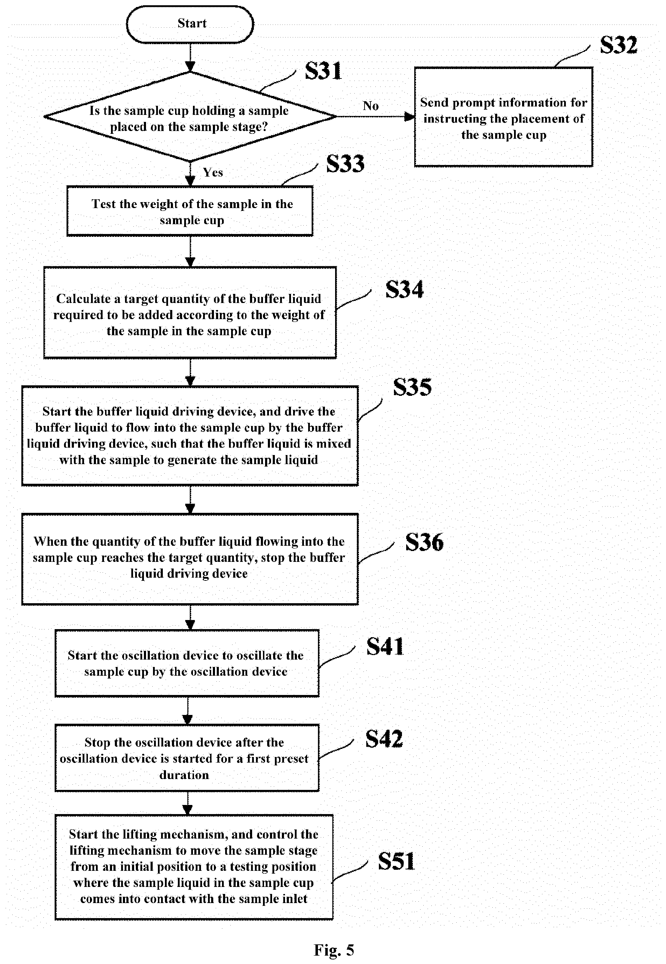

In some embodiments, an oscillation device is further provided on the sample stage 70. Fig. 5 is a schematic flow diagram of the control method according to a fourth embodiment of the present invention. Referring to Fig. 5, after stopping the buffer liquid driving device 30 and before controlling the sample stage 70 to move from an initial position to a testing position thereof, the control method according to the present invention further includes:
step S41: starting the oscillation device to oscillate the sample cup by the oscillation device; and
step S42: stopping the oscillation device after the oscillation device is started for a first preset duration.

Thus, the to-be-tested substance on the sample can be promoted to be fully dissolved into the buffer liquid, so as to form the sample liquid with a proper concentration, thus avoiding the problem that the testing result is inaccurate due to an over low concentration of the sample liquid. Specifically, the first preset duration may be a preset oscillation time which can allow the to-be-tested substance on the sample in the sample cup to be sufficiently dissolved into the buffer liquid according to experimental verification.

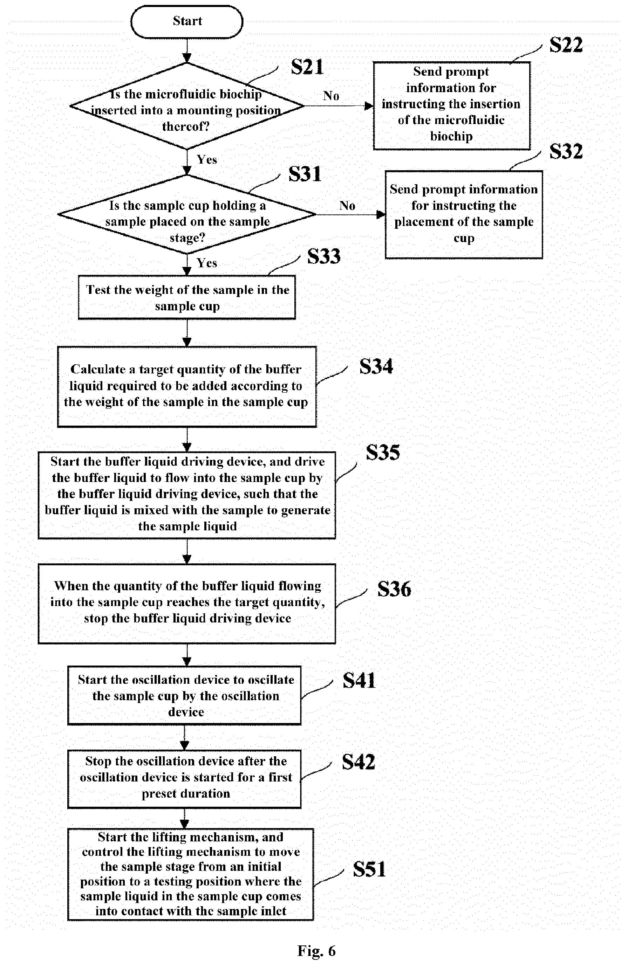

Fig. 6 is a schematic flow diagram of the control method according to a fifth embodiment of the present invention. Referring to Fig. 6, before judging whether the sample cup holding a sample is placed on the sample stage 70, the control method according to the present invention further includes:
step S21: judging whether the microfluidic biochip 10 is inserted into a mounting position thereof; if yes, proceeding to step S31, and if no, proceeding to step S22;
step S22: sending prompt information for instructing the insertion of the microfluidic biochip 10.

That is, in the control method according to the present application, whether the microfluidic biochip 10 is mounted is automatically detected before detecting whether the sample cup is placed on the sample stage 70, and if no, the prompt information is sent to prompt the user to mount the microfluidic biochip 10, such that the user can conveniently and rapidly mount the microfluidic biochip 10 before the sample cup is placed. Specifically, after the microfluidic biochip 10 is mounted to the mounting position thereof, a corresponding trigger switch may be triggered, such that the trigger switch generates a trigger signal for indicating that the microfluidic biochip 10 is mounted in place, and it may be determined that the microfluidic biochip 10 is inserted into the mounting position thereof according to the trigger signal.

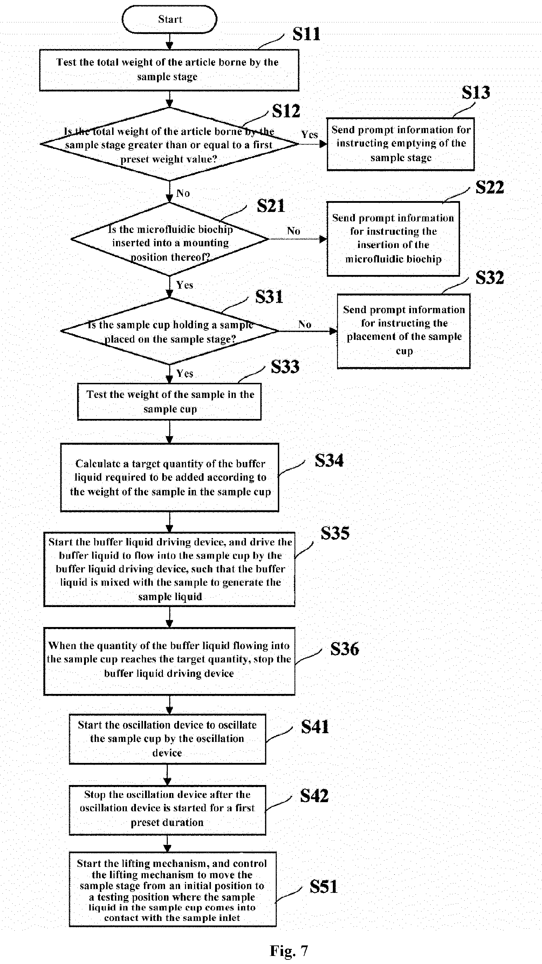

Fig. 7 is a schematic flow diagram of the control method according to a sixth embodiment of the present invention. Referring to Fig. 7, before judging whether the microfluidic biochip 10 is inserted into a mounting position thereof, the control method according to the present invention further includes:
step S11: testing the total weight of the article borne by the sample stage 70;
step S12: judging whether the total weight of the article borne by the sample stage 70 is greater than or equal to a first preset weight value; if yes, proceeding to step S13, and if no, proceeding to step S21; and
step S13: sending prompt information for instructing emptying of the sample stage 70.

That is, in the control method according to the present application, whether other articles (for example, a sample cup left in a previous test or other articles placed on the sample stage by the user) exist on the sample stage is automatically detected before detecting whether the microfluidic biochip 10 is mounted, and if yes, the prompt information for emptying the sample stage is sent out, such that the user can perform operations according to the prompt information, thus improving the automation degree of the microfluidic testing system, and improving the use experience of the user.

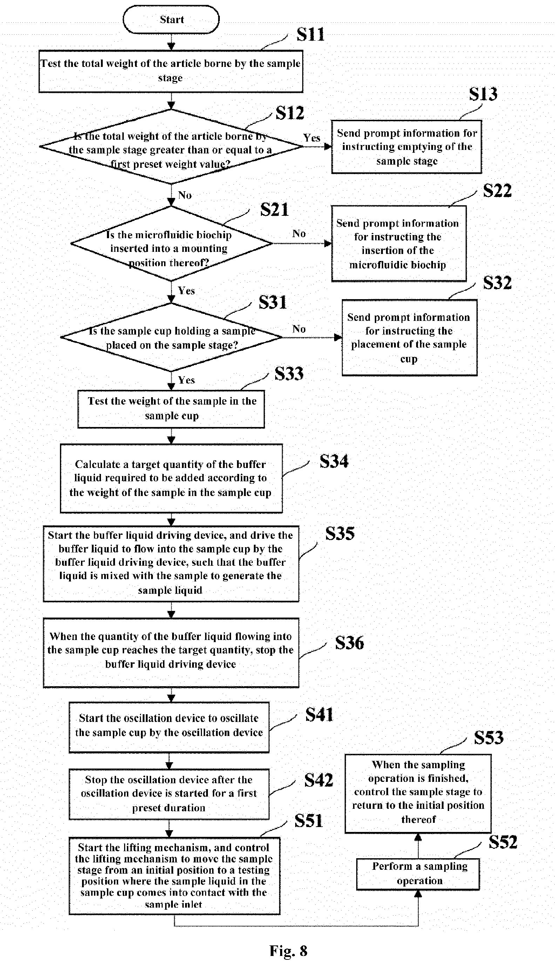

Fig. 8 is a schematic flow diagram of the control method according to a seventh embodiment of the present invention. Referring to Fig. 8, after controlling the sample stage 70 to move from an initial position to a testing position thereof, the control method according to the present invention further includes:
step S52: performing a sampling operation; and
step S53: when the sampling operation is finished, controlling the sample stage 70 to return to the initial position thereof. Thus, an influence on the testing process or the testing result in the microfluidic biochip 10 caused by continuous contact between the sample inlet 111 and the sample liquid may be avoided.

In some embodiments, the microfluidic testing system 1 further includes a sample liquid driving device 40, and the sampling operation of step S52 may specifically include:
driving the sample liquid to flow into the microfluidic biochip 10 through the sample inlet 111 by the sample liquid driving device 40; and
when the quantity of the sample liquid in the microfluidic biochip 10 reaches a preset sample liquid volume value, finishing the sampling operation.

In some embodiments, the sample liquid driving device 40 may form a negative pressure in the microfluidic biochip 10 by pumping air outwards, such that the sample liquid in contact with the sample inlet 111 enters the interior of the microfluidic biochip under the action of the negative pressure. Specifically, the sample liquid driving device 40 may be a micro injection pump, and includes a driving motor, an injector, a lead screw, a slider, a piston, or the like. The quantity of displacement of the piston within the injector is positively correlated to the quantity of the sample liquid entering the microfluidic biochip 10. Therefore, the quantity of the sample liquid entering the microfluidic biochip 10 can be determined by detecting the position of the piston by a position sensor.

In these embodiments, the step of judging whether the quantity of the sample liquid in the microfluidic biochip 10 reaches a preset sample liquid volume value may specifically include:
judging whether a trigger signal for indicating that the piston of the sample liquid driving device moves to a preset position is received; and
if yes, determining that the quantity of the sample liquid in the microfluidic biochip 10 reaches the preset sample liquid volume value. If no, it is considered that the quantity of the sample liquid in the microfluidic biochip 10 does not reach the preset sample liquid volume value.

In the control method according to the present invention, the testing part may be started to be executed after the sampling operation is completed, and the testing part is specifically described below.

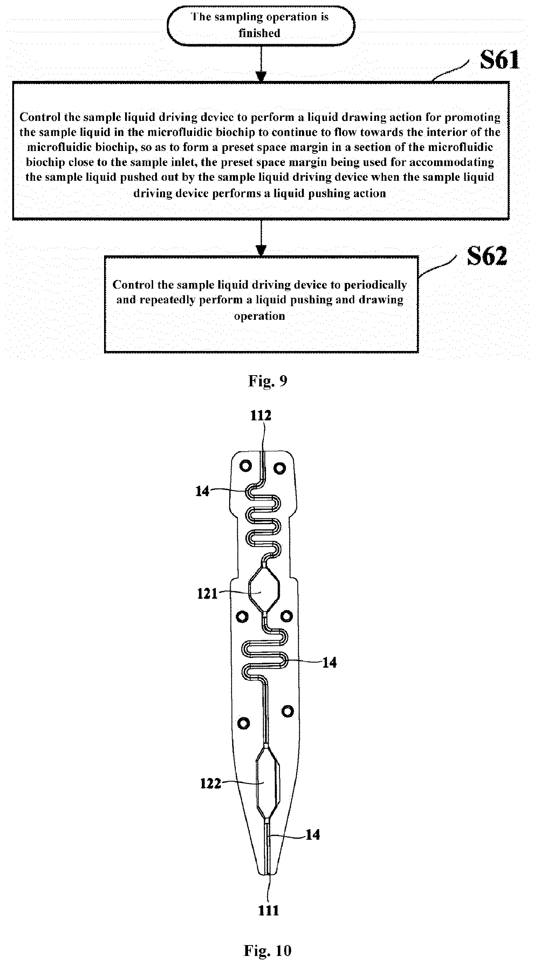

Fig. 9 is a schematic flow diagram of testing of the microfluidic biochip in an embodiment of the present invention. Referring to Fig. 9, in some embodiments, after the sampling operation is finished, the control method according to the present invention further includes:
step S62: controlling the sample liquid driving device 40 to periodically and repeatedly perform a liquid pushing and drawing operation, where the liquid pushing and drawing operation includes a liquid pushing action for promoting the sample liquid in the microfluidic biochip to flow towards the sample inlet 111 and a liquid drawing action for promoting the sample liquid in the microfluidic biochip to flow away from the sample inlet 111.

Since driving forces applied to the sample liquid by the liquid pushing action and the liquid drawing action have opposite directions, the sample liquid can be promoted to repeatedly flow back and forth in the microfluidic biochip 10, thereby facilitating mixing between the sample liquid and a reagent, improving the mixing effect of the sample liquid and the reagent, facilitating a full reaction between the sample liquid and the reagent, and improving the accuracy of the testing result.

Further, after returning the sample stage 70 to the initial position thereof and before controlling the sample liquid driving device to periodically and repeatedly perform a liquid pushing and drawing operation, the control method according to the present invention further includes:
step S61: controlling the sample liquid driving device 40 to perform a liquid drawing action for promoting the sample liquid in the microfluidic biochip 10 to continue to flow towards the interior of the microfluidic biochip, so as to form a preset space margin in a section of the microfluidic biochip 10 close to the sample inlet 111, the preset space margin being used for accommodating the sample liquid pushed out by the sample liquid driving device 40 when the sample liquid driving device 40 performs the liquid pushing action. Thus, the problem of contamination due to a small quantity of the sample liquid being pushed out from the sample inlet 111 when the sample liquid driving device 40 performs the liquid pushing action can be avoided.

Certainly, in other embodiments, the control method according to the present invention may not include step S61, and it is only required to perform the liquid drawing action first and then the liquid pushing action when the sample liquid driving device 40 performs the liquid pushing and drawing operation. Thus, a preset space margin can be also reserved in the section of the microfluidic biochip 10 close to the sample inlet 111.

Fig. 10 is a schematic sectional diagram of the microfluidic biochip suitable to be used according the method of the present invention. The microfluidic biochip 10 has the sample inlet 111 for receiving the sample liquid, a communication port 112 communicated with the sample liquid driving device 40, and a testing pool 121 and a reaction pool 122 formed inside the microfluidic biochip 10, the testing pool 121 being configured to hold a testing reagent, and the reaction pool 122 being configured to hold a reaction reagent. The sample inlet 111, the reaction pool 122, the testing pool 121, and the communication port 112 are sequentially communicated through a micro-channel 14, thereby forming a main channel. For a specific sample liquid or for some specific testing parameters of the sample liquid, the sample liquid may be required to react with the reaction reagent first and then react with the testing reagent, and a testing mechanism tests a solution after the final reaction to obtain preset testing parameters of the specific sample liquid, thus avoiding a reaction or mutual influence between the reaction reagent and the testing reagent, and improving the accuracy of the testing result. For example, when the microfluidic testing system 1 is required to be used to test pesticide residue parameters of the sample liquid, an enzyme inhibition rate method is preferred, and since pesticide residue content is qualitatively tested in the method, the testing speed is higher, and the method is more suitable for household use. At this point, the reaction reagent and the testing reagent for the microfluidic biochip 10 may be an enzyme reagent and a color developing agent respectively. The reaction pool 122 is configured to allow the sample liquid to react with the enzyme reagent therein, and the sample liquid after the reaction with the enzyme reagent flows into the testing pool 121 to react with the color developing agent in the testing pool 121.

In these embodiments, in the sampling operation, the sample liquid driven by the sample liquid driving device 40 flows into the reaction pool 122 through the sample inlet 111, and when the sample liquid flowing into the reaction pool 122 reaches the preset sample liquid volume value, the sampling operation is completed. Meanwhile, the step S62 of controlling the sample liquid driving device 40 to periodically and repeatedly perform a liquid pushing and drawing operation may specifically include:
controlling the sample liquid driving device 40 to periodically and repeatedly perform a second liquid pushing and drawing operation until the number of the second liquid pushing and drawing operations reach a second preset number. The second liquid pushing and drawing operation includes a second liquid pushing action for promoting the sample liquid to flow towards the sample inlet 111 and a second liquid drawing action for promoting the sample liquid to flow towards the reaction pool 122. Thus, mixing of the sample liquid and the reaction reagent is promoted, the mixing effect of the sample liquid and the reaction reagent is improved, and the full reaction between the sample liquid and the reaction reagent is facilitated. The second preset number is a preset number for enabling the sample liquid and the reaction reagent to be uniformly mixed according to experimental verification.

Further, in the same second liquid pushing and drawing operation, the quantity of the sample liquid pushed out by the sample liquid driving device 40 performing the second liquid pushing action is the same as the quantity of the sample liquid drawn in by the sample liquid driving device performing the second liquid drawing action. Thus, the final quantity of the sample liquid in the reaction pool 122 can be guaranteed to be kept unchanged, and it is avoided that due to large error accumulation caused by executing the second liquid pushing and drawing operation multiple times, the quantity of the sample liquid flowing to the testing pool 121 is affected and thus the accuracy of the testing result is affected.

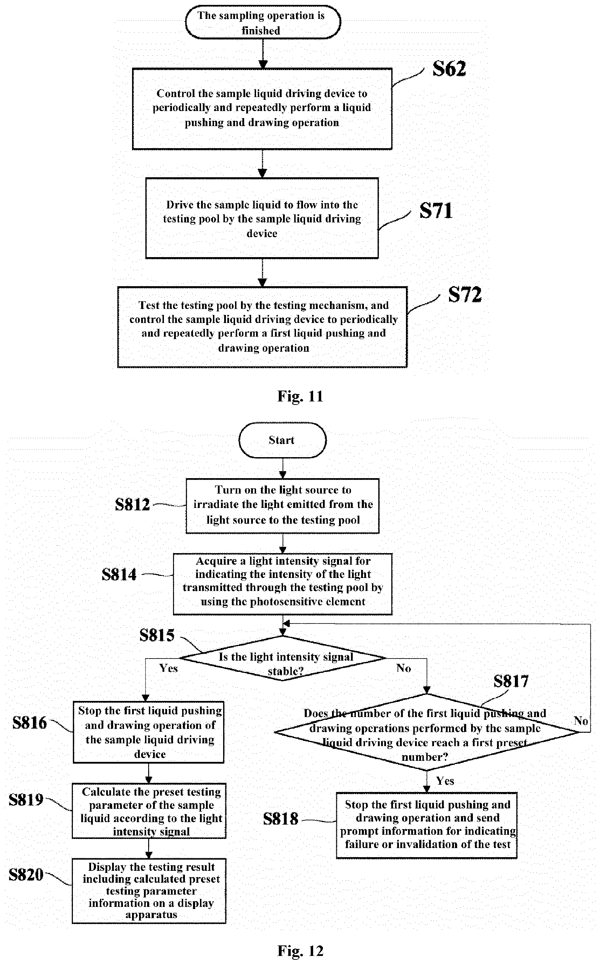

Fig. 11 is a schematic flow diagram of testing of the microfluidic biochip in another embodiment of the present invention. Referring to Fig. 11, in some embodiments, after controlling the sample liquid driving device 40 to periodically and repeatedly perform a second liquid pushing and drawing operation, the control method according to the present invention further includes:
step S71: driving the sample liquid to flow into the testing pool 121 by the sample liquid driving device 40; and
step S72: testing the testing pool 121 by the testing mechanism 20, and controlling the sample liquid driving device 40 to periodically and repeatedly perform a first liquid pushing and drawing operation; where the first liquid pushing and drawing operation includes a first liquid pushing action for promoting the sample liquid to flow out towards the sample inlet 111 and a first liquid drawing action for promoting the sample liquid to flow in towards the testing pool 121.

Since driving forces applied to the sample liquid by the first liquid pushing action and the first liquid drawing action have opposite directions, the sample liquid can be promoted to repeatedly flow back and forth in the microfluidic biochip 10, thereby facilitating mixing between the sample liquid and the testing reagent, improving the mixing effect of the sample liquid and the testing reagent, facilitating the full reaction between the sample liquid and the testing reagent, and improving the accuracy of the testing result.

Further, in the same first liquid pushing and drawing operation, the quantity of the sample liquid pushed out by the sample liquid driving device 40 performing the first liquid pushing action is the same as the quantity of the sample liquid drawn in by the sample liquid driving device performing the first liquid drawing action. Thus, the final quantity of the sample liquid in the testing pool 121 can be guaranteed to be kept unchanged, and it is avoided that due to large error accumulation caused by executing the first liquid pushing and drawing operation multiple times, the accuracy of the testing result is affected.

Fig. 12 is a schematic flow diagram of testing of the testing pool by the testing mechanism in an embodiment of the present invention. In some embodiments, the testing mechanism 20 may include a light source and a photosensitive element arranged on two opposite sides of the testing pool 121 respectively; a step of testing the testing pool 121 by the testing mechanism 20 includes:
step S812: turning on the light source to irradiate the light emitted from the light source to the testing pool 121;
step S814: acquiring a light intensity signal for indicating the intensity of the light transmitted through the testing pool 121 by using the photosensitive element;
step S815: judging whether the light intensity signal is stable; if yes, proceeding to step S816, and if no, proceeding to step S817;
step S816: stopping the first liquid pushing and drawing operation of the sample liquid driving device 40, and proceeding to step S819;
step S817: judging whether the number of the first liquid pushing and drawing operations performed by the sample liquid driving device 40 reaches a first preset number; if yes, proceeding to step S818; if no, proceeding to step S815 to continue to judge the stability of the light intensity signal;
step S818: stopping the first liquid pushing and drawing operation and sending prompt information for indicating failure or invalidation of the test; and
step S819: calculating the preset testing parameter of the sample liquid according to the light intensity signal.

Taking the testing of the pesticide residue parameter of the sample liquid by the microfluidic testing system 1 as an example, the reaction reagent held in the reaction pool 122 may be an enzyme reagent, and the testing reagent held in the testing pool 121 may be a color developing agent. After the sample liquid enters the reaction pool 122, pesticide residues in the sample liquid react with the enzyme reagent using the principle that a pesticide may inhibit the activity of enzyme. The solution after the reaction enters the testing pool 121. Light emitted from the light source 21 is irradiated to the testing pool 121, and light transmitted through the testing pool 121 is introduced into the photosensitive element 22, which facilitates judgment of the change in an absorbance in the testing pool 121 using the stable light intensity signal received by the photosensitive element 22, and then facilitates calculation of a pesticide residue inhibition rate.

It may be understood that the relatively stable light intensity signal can be received only after the sample liquid and the testing reagent fully react, and the preset testing parameter of the sample liquid calculated according to the stable light intensity signal is relatively accurate. Therefore, whether the sample liquid and the testing reagent fully react can be judged by judging whether the light intensity signal is stable. If the sample liquid and the testing reagent have fully reacted, the sample liquid driving device 40 is not required to go on performing the first liquid pushing and drawing operation, and the first liquid pushing and drawing operation may be stopped in time to reduce energy consumption. If the relatively stable light intensity signal is not received yet after the number of the first liquid pushing and drawing operations reaches the first preset number, the sample liquid and the testing reagent may not fully react, for example, the testing reagent loses efficacy or other reasons occur; at this point, timely stopping of the first liquid pushing and drawing operation may reduce energy consumption, prompt information is sent to remind the user that this test is invalid or fails, and the user can conveniently make corresponding measures in time.

In some embodiments, the control method according to the present invention further includes:
after receiving a trigger signal for instructing starting of the testing function of the microfluidic testing system, starting a heating module of the microfluidic testing system, and heating the testing pool 121 by the heating module;
acquiring the temperature of the testing pool 121 periodically or in real time; and
stopping the heating module when the temperature of the testing pool 121 is higher than a preset upper temperature limit value, and restarting the heating module when the temperature in the testing pool 121 is lower than a preset lower temperature limit value.

Thus, the testing pool 121 may be guaranteed to always have a relatively constant temperature before a testing operation is performed, thus facilitating the sufficient reaction of the sample liquid and the testing reagent.

In some embodiments, after stopping the first liquid pushing and drawing operation of the sample liquid driving device 40, the control method according to the present invention further includes:
turning off the light source and stopping the heating module. This step may occur before or after step S819.

It should be noted that the starting operation of the heating module, the temperature acquisition of the heating module, and the temperature control of the heating module are continuously performed during the whole testing process, so as to ensure that the testing pool 121 has a relatively constant temperature range all the time during the whole testing process.

In some embodiments, after the step S819 of calculating the preset testing parameter of the sample liquid according to the light intensity signal, the control method according to the present invention further includes:
step S820: displaying the testing result including calculated preset testing parameter information on a display apparatus. The display apparatus may be, for example, a display screen, or a color indicator lamp, or include both a display screen and a color indicator lamp.

The microfluidic testing system 1 may include a microfluidic biochip 10 for providing testing conditions and testing environments, a sample stage 70 for placing a sample cup, a lifting mechanism 60 for driving the sample stage 70 to move, a sample liquid driving device 40 for driving a sample liquid to flow, a buffer liquid driving device 30 for driving a buffer liquid to flow into the sample cup, a testing mechanism 20 for performing a testing operation, and a buffer liquid bottle 36 for storing the buffer liquid. The sample stage 70 may be located below the microfluidic biochip 10, such that the sample liquid in the sample cup thereon is in contact with a sample inlet 111 located at the bottom of the microfluidic biochip 10. The lifting mechanism 60 is adjacently provided on a transverse side of the sample stage 70, so as to drive the sample stage 70 to move up and down. The buffer liquid driving device 30 may be provided on one side of the microfluidic biochip 10 in the transverse direction and located above the lifting mechanism 60, the sample liquid driving device 40 may be provided on the other side of the microfluidic biochip 10 in the transverse direction, and the buffer liquid bottle 36 is located on the side of the sample liquid driving device 40 away from the microfluidic biochip 10. Thus, the size features of each module in the vertical direction and the transverse direction can be fully utilized, such that the layout of the modules is more compact, and the occupied space is reduced as far as possible. Moreover, the modules are only arranged side by side in the vertical direction and the transverse direction, such that the thickness of the microfluidic testing system 1 in the front and rear direction is reduced as far as possible, and the microfluidic testing system is more suitable for being integrated on a refrigerator.

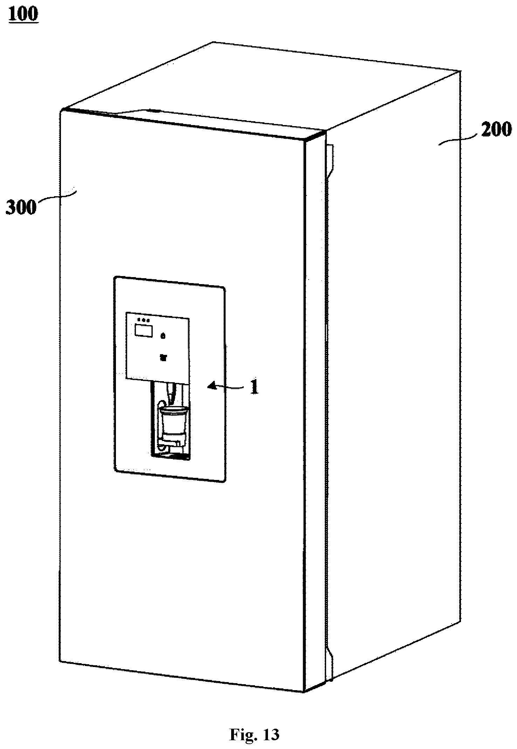

Fig. 13 is a schematic structural diagram of the refrigerator; the refrigerator 100 includes the microfluidic testing system 1 so as to integrate the microfluidic testing system 1 on the refrigerator 100. The refrigerator 100 is frequently used in daily life, and mainly configured to store food materials, and when the microfluidic testing system 1 is integrated on the refrigerator 100, a user can conveniently perform a testing operation of a food material sample by using the microfluidic testing system 1.

Further, the refrigerator 100 further includes a cabinet 200 and a door 300, the cabinet 200 defines a storage space therein, and the door 300 is connected to the cabinet 200 and configured to open and/or close the storage space. The microfluidic testing system 1 is preferably provided on the door 300, such that the operation is convenient, an original storage space in the cabinet 200 cannot be occupied, and the storage capacity of the refrigerator 100 cannot be influenced. The microfluidic testing system 1 may be electrically connected to an electrical control device of the refrigerator 100, so as to provide power for the microfluidic testing system 1 by the electrical control device and/or to allow signals to be transmitted between the electrical control device and the microfluidic testing system 1.

The refrigerator 100 is a refrigerator in a broad sense, and includes not only a so-called refrigerator in a narrow sense, but also a storage device having a refrigerating, freezing or other storage functions, for example, a refrigerating box, a freezer, or the like.

## Claims

1. A control method for a microfluidic testing system, the microfluidic testing system comprising a microfluidic biochip (10), a sample stage (70) for placing a sample cup (2), and a lifting mechanism (60) for driving the sample stage (70) to move, the microfluidic biochip (10) being provided with a sample inlet (111) for receiving a sample liquid, and the control method comprising:
starting the lifting mechanism (60) after the sample cup (2) holding the sample liquid is placed on the sample stage (70), and controlling the lifting mechanism (60) to move the sample stage (70) from an initial position to a testing position where the sample liquid in the sample cup (2) comes into contact with the sample inlet (111), wherein the microfluidic testing system further comprises a buffer liquid driving device (30), and wherein before starting the lifting mechanism (60), the control method further comprises a step of judging whether the sample cup (2) holding the sample liquid is placed on the sample stage (70), and the step specifically comprises:
judging whether the sample cup (2) holding a sample is placed on the sample stage (70); and
if yes, starting the buffer liquid driving device (30), and driving a buffer liquid to flow into the sample cup (2) by the buffer liquid driving device (30), such that the buffer liquid is mixed with the sample in the sample cup (2) to generate the sample liquid.

2. The control method according to claim 1,
wherein after the sample cup (2) holding the sample is placed on the sample stage (70) and before the buffer liquid driving device (30) is started, the control method further comprises:
testing the weight of the sample in the sample cup (2); and
calculating a target quantity of the buffer liquid required to be added according to the weight of the sample in the sample cup (2); and
after starting the buffer liquid driving device (30), the control method further comprises:
when the quantity of the buffer liquid flowing into the sample cup (2) reaches the target quantity, stopping the buffer liquid driving device (30).

3. The control method according to claim 2, wherein an oscillation device is further provided on the sample stage (70), and after stopping the buffer liquid driving device (30) and before controlling the sample stage (70) to move from an initial position to a testing position thereof, the control method further comprises:
starting the oscillation device to oscillate the sample cup (2) by the oscillation device; and
stopping the oscillation device after the oscillation device is started for a first preset duration.

4. The control method according to any one of claims 1 to 3, wherein before judging whether the sample cup (2) holding a sample is placed on the sample stage (70), the control method further comprises:
judging whether the microfluidic biochip (10) is inserted into a mounting position thereof; and
if yes, judging whether the sample cup (2) holding the sample is placed on the sample stage (70), and if no, sending prompt information for instructing the insertion of the microfluidic biochip (10).

5. The control method according to claim 4, wherein before judging whether the microfluidic biochip (10) is inserted into a mounting position thereof, the control method further comprises:
testing the total weight of the article borne by the sample stage (70); and
when the total weight of the article borne by the sample stage (70) is greater than or equal to a first preset weight value, sending prompt information for instructing emptying of the sample stage (70); and when the total weight of the article borne by the sample stage (70) is less than the first preset weight value, judging whether the microfluidic biochip (10) is inserted into the mounting position thereof.

6. The control method according to any one of claims 1 to 5, wherein after controlling the sample stage (70) to move from an initial position to a testing position thereof, the control method further comprises:
performing a sampling operation; and
when the sampling operation is finished, controlling the sample stage (70) to return to the initial position thereof.

7. The control method according to claim 6, wherein the microfluidic testing system further comprises a sample liquid driving device (40), and the sampling operation comprises:
driving the sample liquid to flow into the microfluidic biochip (10) through the sample inlet (111) by the sample liquid driving device (40); and
when the quantity of the sample liquid in the microfluidic biochip (10) reaches a preset sample liquid volume value, finishing the sampling operation.

8. The control method according to claim 7, wherein the sample liquid driving device (40) is a micro injection pump, and
the step of judging whether the quantity of the sample liquid in the microfluidic biochip (10) reaches a preset sample liquid volume value comprises:
judging whether a trigger signal for indicating that a piston of the sample liquid driving device (40) moves to a preset position is received; and
if yes, determining that the quantity of the sample liquid in the microfluidic biochip (10) reaches the preset sample liquid volume value.

9. The control method according to claim 8 or 7, wherein after the sampling operation is finished, the control method further comprises:
controlling the sample liquid driving device (40) to periodically and repeatedly perform a liquid pushing and drawing operation, the liquid pushing and drawing operation comprising a liquid pushing action for promoting the sample liquid in the microfluidic biochip (10) to flow towards the sample inlet (111) and a liquid drawing action for promoting the sample liquid in the microfluidic biochip (10) to flow away from the sample inlet (111).

10. The control method according to claim 9, wherein after the sample stage (70) returning to the initial position thereof and before controlling the sample liquid driving device (40) to periodically and repeatedly perform a liquid pushing and drawing operation, the control method further comprises:
controlling the sample liquid driving device (40) to perform a liquid drawing action for promoting the sample liquid in the microfluidic biochip (10) to continue to flow towards the interior of the microfluidic biochip (20), so as to form a preset space margin in a section of the microfluidic biochip (10) close to the sample inlet (111), the preset space margin being used for accommodating the sample liquid pushed out by the sample liquid driving device (40) when the sample liquid driving device (40) performs the liquid pushing action.

## Patentansprüche

1. Steuerverfahren für ein mikrofluidisches Prüfsystem, wobei das mikrofluidisches Prüfsystem einen mikrofluidischen Biochip (10), einen Probentisch (70) zum Platzieren eines Probenbechers (2) und einen Hebemechanismus (60) zum Antrieb des Probentisches (70) aufweist, wobei der mikrofluidisches Biochip (10) mit einem Probeneinlass (111) zum Empfangen einer Probenflüssigkeit versehen ist, wobei das Steuerverfahren umfasst:
Starten des Hubmechanismus (60), nachdem der Probenbecher (2), der die Probenflüssigkeit enthält, auf den Probentisch (70) platziert ist, und Steuern des Hubmechanismus (60), um den Probentisch (70) von einer Ausgangsposition in eine Prüfposition zu bewegen, in der die Probenflüssigkeit im Probenbecher (2) mit dem Probeneinlass (111) in Berührung kommt, wobei das mikrofluidische Prüfsystem weiterhin eine Pufferflüssigkeitsantriebsvorrichtung (30) aufweist und wobei vor dem Starten des Hubmechanismus (60), das Steuerverfahren ferner einen Schritt des Beurteilens, ob der Probenbecher (2), der die Probenflüssigkeit enthält, auf den Probentisch (70) platziert wird, umfasst, und wobei der Schritt insbesondere umfasst:
Feststellen, ob der Probenbecher (2), der eine Probe enthält, auf den Probentisch (70) platziert wird; und
wenn ja, Starten der Pufferflüssigkeitsantriebsvorrichtung (30) und Treiben einer Pufferflüssigkeit durch die Pufferflüssigkeitsantriebsvorrichtung (30) zum Fluss in den Probenbecher (2), so dass die Pufferflüssigkeit mit der Probe in dem Probenbecher (2) gemischt wird, um die Probenflüssigkeit zu erzeugen.

2. Steuerverfahren gemäß Anspruch 1,
wobei nach dem Platzieren des Probenbechers (2), der die Probe enthält, auf den Probentisch (70) und vor dem Starten der Pufferflüssigkeitsantriebsvorrichtung (30) das Steuerverfahren ferner umfasst:
Prüfen des Gewichts der Probe im Probenbecher (2); und
Berechnen der Zielmenge der zugefügten Pufferflüssigkeit entsprechend dem Gewicht der Probe im Probenbecher (2); und
nach dem Starten der Pufferflüssigkeitsantriebsvorrichtung (30), das Steuerverfahren ferner umfasst:
Stoppen der Pufferflüssigkeitsantriebsvorrichtung (30), wenn die Menge der Pufferflüssigkeit, die in den Probenbecher (2) fließt, die Zielmenge erreicht.

3. Steuerverfahren gemäß Anspruch 2, wobei auf dem Probentisch (70) weiterhin eine Schwingungsvorrichtung vorgesehen ist und nach dem Stoppen der Pufferflüssigkeitsantriebsvorrichtung (30) und vor dem Steuern des Probentisches (70), um sich von einer Ausgangsposition in eine Prüfposition zu bewegen, das Steuerverfahren weiterhin umfasst:
Starten der Schwingungsvorrichtung zum Schwingen des Probenbechers (2) durch die Schwingungsvorrichtung; und
Stoppen der Schwingvorrichtung nach dem Starten der Schwingvorrichtung für eine erste voreingestellte Dauer.

4. Steuerverfahren gemäß einem der Ansprüche 1 bis 3, wobei vor des Beurteilens, ob der Probenbecher (2), der eine Probe enthält, auf den Probentisch (70) platziert ist, das Steuerverfahren ferner umfasst:
Feststellen, ob der mikrofluidische Biochip (10) in dessen Montageposition eingesetzt ist; und
wenn ja, Beurteilen, ob der Probenbecher (2), der die Probe enthält, auf dem Probentisch (70) platziert ist, und wenn nicht, Senden eine Aufforderungsinformation zur Anweisung des Einfügens des mikrofluidischen Biochips (10).

5. Steuerverfahren gemäß Anspruch 4, wobei vor des Beurteilens, ob der mikrofluidische Biochip (10) in dessen Montageposition eingesetzt ist, das Steuerverfahren ferner umfasst:
Prüfen des Gesamtgewichts des von dem Probentisch (70) getragenen Gegenständes; und
Senden eine Aufforderungsinformation zur Anweisung der Entleerung des Probentisches (70), wenn das Gesamtgewicht des von dem Probentisch (70) getragenen Gegenständes größer oder gleich einem ersten voreingestellten Gewichtswert ist; und Feststellen, ob der mikrofluidische Biochip (10) in dessen Montageposition eingesetzt ist, wenn das Gesamtgewicht des von dem Probentisch (70) getragenen Gegenständes kleiner als der erste voreingestellte Gewichtswert ist.

6. Steuerverfahren nach einem der Ansprüche 1 bis 5, wobei nach dem Steuern der Bewegung des Probentisches (70) von seiner Ausgangsposition in seine Prüfposition, das Steuerverfahren ferner umfasst:
Durchführen eines Probenahmevorgangs; und
Steuern des Probentisches (70), um in seine Ausgangsposition zurückzukehren, wenn der Probenahmevorgang endet ist.

7. Steuerverfahren gemäß Anspruch 6, wobei das mikrofluidische Prüfsystem weiterhin eine Probenflüssigkeitsantriebsvorrichtung (40) umfasst und wobei der Probenahmevorgang umfasst:
Antreiben der Probenflüssigkeit durch die Probeneinlass (111) in den mikrofluidischen Biochip (10) durch die Probenflüssigkeitsantriebsvorrichtung (40); und
Enden des Probenahmevorgangs, wenn die Menge der Probenflüssigkeit im mikrofluidischen Biochip (10) einen voreingestellten Probenflüssigkeitsvolumewert erreicht.

8. Steuerverfahren gemäß Anspruch 7, wobei die Probenflüssigkeitsantriebsvorrichtung (40) eine Mikroinjektionspumpe ist, und
der Schritt des Beurteilens, ob die Menge der Probenflüssigkeit im mikrofluidischen Biochip (10) einen voreingestellten Probenflüssigkeitsvolumewert erreicht, umfasst:
Beurteilen, ob ein Auslösesignal empfangen wird, das anzeigt, dass sich ein Kolben der Probenflüssigkeitsantriebsvorrichtung (40) in eine voreingestellte Position bewegt; und
Wenn ja, Bestimmen, dass die Menge der Probenflüssigkeit in dem mikrofluidischen Biochip (10) den voreingestellten Probenflüssigkeitsvolumenwert erreicht.

9. Steuerverfahren gemäß Anspruch 8 oder 7, wobei nach Enden des Probenahmevorgangs, das Steuerverfahren ferner umfasst:
Steuern der Probenflüssigkeitsantriebsvorrichtung (40), um periodisch und wiederholt einen Flüssigkeitsschieb- und -ziehvorgang durchzuführen, wobei der Flüssigkeitsschieb- und -ziehvorgang einen Flüssigkeitsschiebvorgang zum Fördern der Probenflüssigkeit im mikrofluidischen Biochip (10) zum Fluss in Richtung des Probeneinlasses (111) und einen Flüssigkeitsziehvorgang zum Fördern der Probenflüssigkeit im mikrofluidischen Biochip (10) zum Fluss vom Probeneinlass (111) umfasst.

10. Steuerverfahren gemäß Anspruch 9, wobei nach dem Rückkehren des Probentisches (70) in seine Ausgangsposition und vor dem Steuern der Probenflüssigkeitsantriebsvorrichtung (40) zur regelmäßigen und wiederholten Durchführung eines Flüssigkeitsschieb- und -ziehvorgangs, das Steuerverfahren ferner umfasst:
Steuern der Probenflüssigkeitsantriebsvorrichtung (40), um einen Flüssigkeitsziehvorgang auszuführen, um die Probenflüssigkeit im mikrofluidischen Biochip (10) weiter in Richtung des mikrofluidischen Biochips (20) zu fließen, so dass in einem Abschnitt des mikrofluidischen Biochips (10) nahe dem Probeneinlass (111) eine voreingestellte Raumspanne gebildet wird, die zur Aufnahme der von der Probenflüssigkeitsantriebsvorrichtung (40) ausgestockten Probenflüssigkeit verwendet wird, wenn die Probenflüssigkeitsantriebsvorrichtung (40) die Flüssigkeitsschiebvorgang ausführt.

## Revendications

1. Méthode de contrôle pour un système de test microfluidique, le système de test microfluidique comprenant une biopuce microfluidique (10), un étage d'échantillonnage (70) pour placer une coupelle d'échantillonnage (2), et un mécanisme de levage (60) pour entraîner le déplacement de l'étage d'échantillonnage (70), la biopuce microfluidique (10) étant pourvue d'une entrée d'échantillon (111) pour recevoir un échantillon liquide, et la méthode de contrôle comprenant :
le démarrage du mécanisme de levage (60) après que la coupelle d'échantillonnage (2) contenant l'échantillon liquide est placée sur l'étage d'échantillonnage (70), et la commande du mécanisme de levage (60) pour déplacer l'étage d'échantillonnage (70) d'une position initiale à une position d'essai dans laquelle l'échantillon liquide dans la coupelle d'échantillonnage (2) entre en contact avec l'entrée d'échantillon (111), dans lequel le système de test microfluidique comprend en outre un dispositif d'entraînement du liquide tampon (30), et dans lequel, avant de démarrer le mécanisme de levage (60), la méthode de contrôle comprend en outre une étape consistant à juger si la coupelle d'échantillonnage (2) contenant l'échantillon liquide est placée sur l'étage d'échantillonnage (70), et l'étape comprend en particulier :
juger si la coupelle d'échantillonnage (2) contenant un échantillon est placé sur l'étage d'échantillonnage (70) ; et
dans l'affirmative, démarrer le dispositif d'entraînement du liquide tampon (30) et faire couler un liquide tampon dans la coupelle d'échantillonnage (2) par le dispositif d'entraînement du liquide tampon (30), de sorte que le liquide tampon soit mélangé à l'échantillon dans la coupelle d'échantillonnage (2) pour générer l'échantillon liquide.

2. Méthode de contrôle selon la revendication 1,
dans laquelle, après que la coupelle d'échantillonnage (2) contenant l'échantillon a été placée sur l'étage d'échantillonnage (70) et avant que le dispositif d'entraînement du liquide tampon (30) ne soit mis en marche, la méthode de contrôle comprend en outre :
tester le poids de l'échantillon dans la coupelle d'échantillonnage (2) ; et
calculer une quantité cible de liquide tampon à ajouter en fonction du poids de l'échantillon dans la coupelle d'échantillonnage (2) ; et
après avoir démarré le dispositif d'entraînement du liquide tampon (30), la méthode de contrôle comprend en outre :
lorsque la quantité de liquide tampon s'écoulant dans la coupelle d'échantillonnage (2) atteint la quantité cible, arrêter le dispositif d'entraînement du liquide tampon (30).

3. Méthode de contrôle selon la revendication 2, dans laquelle un dispositif d'oscillation est également prévu sur l'étage d'échantillonnage (70), et après avoir arrêté le dispositif d'entraînement du liquide tampon (30) et avant de commander l'étage d'échantillonnage (70) pour la faire passer d'une position initiale à une position d'essai, la méthode de contrôle comprend en outre :
démarrer le dispositif d'oscillation pour faire osciller la coupelle d'échantillonnage (2) à l'aide du dispositif d'oscillation ; et
arrêter le dispositif d'oscillation après le démarrage du dispositif d'oscillation pendant une première durée prédéfinie.

4. Méthode de contrôle selon l'une quelconque des revendications 1 à 3, dans laquelle avant de juger si la coupelle d'échantillonnage (2) contenant un échantillon est placée sur l'étage d'échantillonnage (70), la méthode de contrôle comprend en outre :
juger si la biopuce microfluidique (10) est insérée dans une position de montage de celle-ci ; et
dans l'affirmative, déterminer si la coupelle d'échantillonnage (2) contenant l'échantillon est placée sur l'étage d'échantillonnage (70) et, dans la négative, envoyer une information rapide pour ordonner l'insertion de la biopuce microfluidique (10).

5. Méthode de contrôle selon la revendication 4, dans laquelle avant de juger si la biopuce microfluidique (10) est insérée dans une position de montage, la méthode de contrôle comprend en outre :
tester le poids total de l'article porté par l'étage d'échantillonnage (70) ; et
lorsque le poids total de l'article porté par l'étage d'échantillonnage (70) est supérieur ou égal à une première valeur de poids prédéfinie, envoyer une information rapide pour demander la vidange de l'étage d'échantillonnage (70) ; et lorsque le poids total de l'article porté par l'étage d'échantillonnage (70) est inférieur à la première valeur de poids prédéfinie, juger si la biopuce microfluidique (10) est insérée dans la position de montage de cette dernière.

6. Méthode de contrôle selon l'une quelconque des revendications 1 à 5, dans laquelle, après avoir commandé le déplacement de l'étage d'échantillonnage (70) d'une position initiale à une position d'essai, la méthode de contrôle comprend en outre :
effectuer une opération d'échantillonnage ; et
lorsque l'opération d'échantillonnage est terminée, commander l'étage d'échantillonnage (70) pour qu'il revienne à sa position initiale.

7. Méthode de contrôle selon la revendication 6, dans laquelle le système d'essai microfluidique comprend en outre un dispositif d'entraînement de l'échantillon liquide (40), et l'opération d'échantillonnage comprend :
le dispositif d'entraînement de l'échantillon liquide (40) fait circuler l'échantillon liquide dans la biopuce microfluidique (10) à travers l'entrée d'échantillon (111) ; et
lorsque la quantité d'échantillon liquide dans la biopuce microfluidique (10) atteint une valeur prédéfinie de volume d'échantillon liquide, terminer l'opération d'échantillonnage.

8. Méthode de contrôle selon la revendication 7, dans laquelle le dispositif d'entraînement de l'échantillon liquide (40) est une pompe à micro-injection, et
l'étape consistant à déterminer si la quantité de l'échantillon liquide dans la biopuce microfluidique (10) atteint une valeur prédéfinie de volume de l'échantillon liquide comprend :
juger si un signal de déclenchement indiquant qu'un piston du dispositif d'entraînement de l'échantillon liquide (40) se déplace jusqu'à une position prédéfinie est reçu ; et
dans l'affirmative, déterminer que la quantité d'échantillon liquide dans la biopuce microfluidique (10) atteint la valeur prédéfinie du volume d'échantillon liquide.

9. Méthode de contrôle selon la revendication 8 ou 7, dans laquelle, une fois l'opération d'échantillonnage terminée, la méthode de contrôle comprend en outre :
commander le dispositif d'entraînement de l'échantillon liquide (40) pour effectuer périodiquement et de manière répétée une opération de poussée et d'aspiration du liquide, l'opération de poussée et d'aspiration du liquide comprenant une action de poussée du liquide pour favoriser l'écoulement de l'échantillon liquide dans la biopuce microfluidique (10) vers l'entrée d'échantillon (111) et une action d'aspiration du liquide pour favoriser l'écoulement de l'échantillon liquide dans la biopuce microfluidique (10) loin de l'entrée d'échantillon (111).

10. Méthode de contrôle selon la revendication 9, dans laquelle après le retour de l'étage d'échantillonnage (70) à sa position initiale et avant de commander le dispositif d'entraînement de l'échantillon liquide (40) pour effectuer périodiquement et de manière répétée une opération de poussée et d'aspiration du liquide, la méthode de contrôle comprend en outre :
la commande du dispositif d'entraînement de l'échantillon liquide (40) pour effectuer une action d'aspiration du liquide afin de promouvoir l'échantillon liquide dans la biopuce microfluidique (10) pour continuer à s'écouler vers l'intérieur de la biopuce microfluidique (20), de manière à former une marge d'espace prédéfinie dans une section de la biopuce microfluidique (10) proche de l'entrée d'échantillon (111), la marge d'espace prédéfinie étant utilisée pour accueillir l'échantillon liquide poussé par le dispositif d'entraînement de l'échantillon liquide (40) lorsque le dispositif d'entraînement de l'échantillon liquide (40) effectue l'action de poussée du liquide.
